# EUROPÄISCHE PATENTSCHRIFT

(11) **EP 2 231 117 B1**
(45) Veröffentlichungstag und Bekanntmachung des Hinweises auf die Patenterteilung: **14.10.2015**
(21) Anmeldenummer: 09702850.0
(22) Anmeldetag: 15.01.2009
(51) Int. Cl.: A61K 9/08, A61K 31/55

(54) **BENZODIAZEPIN UND/ODER BENZODIAZEPINDERIVATE ENTHALTENDE PHARMAZEUTISCHE ZUSAMMENSETZUNG**
BENZODIAZEPINE AND/OR PHARMACEUTICAL COMPOSITION COMPRISING BENZODIAZAPINE DERIVATIVES
COMPOSITION PHARMACEUTIQUE COMPRENANT UNE BENZODIAZÉPINE ET/OU DES DÉRIVÉS DE BENZODIAZÉPINE

(30) Priorität: 16.01.2008 DE 102008004694
(43) Veröffentlichungstag der Anmeldung: 29.09.2010
(73) Patentinhaber: Dr. Franz Köhler Chemie GmbH, 64625 Bensheim (DE)
(72) Erfinder: HOERNECKE, Rainer, 80999 München (DE)
(74) Vertreter: Becker Kurig Straus
(86) Internationale Anmeldenummer: PCT/DE2009/000042
(87) Internationale Veröffentlichungsnummer: WO 2009/089826

(56) Entgegenhaltungen:
- US-A- 4 832 952
- BITTNER B ET AL: "Impact of Solutol HS 15 on the pharmacokinetic behavior of midazolam upon intravenous administration to male Wistar rats" EUROPEAN JOURNAL OF PHARMACEUTICS AND BIOPHARMACEUTICS, ELSEVIER SCIENCE PUBLISHERS B.V., AMSTERDAM, NL, Bd. 56, Nr. 1, 1. Juli 2003 (2003-07-01), Seiten 143-146, XP004434930 ISSN: 0939-6411
- COLPAERT F C ET AL: "Discriminative stimulus properties of benzodiazepines, barbiturates and pharmacologically related drugs; Relation to some intrinsic and anticonvulsant effects" EUROPEAN JOURNAL OF PHARMACOLOGY, ELSEVIER BV, NL, Bd. 37, Nr. 1, 1. Mai 1976 (1976-05-01), Seiten 113-123, XP025812190 ISSN: 0014-2999 [gefunden am 1976-05-01]
- MENON M ET AL: "Serotonergic drugs, benzodiazepines and baclofen block muscimol-induced myoclonic jerks in a strain of mice" EUROPEAN JOURNAL OF PHARMACOLOGY, ELSEVIER BV, NL, Bd. 73, Nr. 2-3, 17. Juli 1981 (1981-07-17), Seiten 155-161, XP025558242 ISSN: 0014-2999 [gefunden am 1981-07-17]
- SIMIAND J ET AL: "Comparative study in mice of tetrazepam and other centrally active skeletal muscle relaxants." ARCHIVES INTERNATIONALES DE PHARMACODYNAMIE ET DE THÉRAPIE 1989 JAN-FEB, Bd. 297, Januar 1989 (1989-01), Seiten 272-285, XP009116721 ISSN: 0301-4533
- ZARRINDAST M R ET AL: "Involvement of dopaminergic receptor subtypes in straub tail behaviour in mice" GENERAL PHARMACOLOGY, PERGAMON PRESS, OXFORD, GB, Bd. 24, Nr. 1, 1. Januar 1993 (1993-01-01), Seiten 127-130, XP023819124 ISSN: 0306-3623 [gefunden am 1993-01-01]

## Beschreibung

Die Erfindung betrifft eine das Benzodiazepin Lormetazepam und/oder Lormetazepamderivate oder pharmazeutisch verträgliche Salze davon enthaltende pharmazeutische Zusammensetzung. Ferner betrifft die Erfindung diese pharmazeutische Zusammensetzung zur Verwendung als Arzneimittel zur Induktion einer opiatanalogen Wirkung, und sie betrifft ein Verfahren zum Herstellen einer entsprechenden Injektions- oder Infusionslösung.

Lorazepam (INN) ist ein 7-Chloro-5-(o-chlorophenyl)-1,3-dihydro-3-hydroxy-2H-1,4-benzodiazepin-2-on (WHO) bzw. 9-Chloro-6-(2-chlorophenyl)-4-hydroxy-2,5-diazabicyclo[5.4.0]undeca-5,8,10,12-tetraen-3-on (systematischer Name der IUPAC).

Lorazepam ist eine psychotrope Substanz aus der Klasse der 1,4-Benzodiazepine mit spannungs-, erregungs- und angstdämpfenden Eigenschaften sowie sedierenden und hypnotischen Effekten. Darüber hinaus zeigt Lorazepam den Muskeltonus dämpfende und antikonvulsive Wirkungen.

Lorazepam besitzt eine sehr hohe Affinität zu spezifischen Bindungsstellen im Zentralnervensystem. Diese Bindungsstellen stehen in enger funktioneller Verbindung mit den Rezeptoren des inhibitorischen Neurotransmitters Gamma-Aminobuttersäure (GABA). Nach Bindung an den Rezeptor verstärkt Lorazepam die GABAerge Hemmung der Aktivität nachgeschalteter Neurone.

Intravenös und intramuskulär verabreichte lorazepamhaltige therapeutische Zusammensetzungen sind seit 1988 in Deutschland zur Behandlung zugelassen zur Basissedierung vor und während diagnostischer und operativer Eingriffe zugelassen, um Angst und Spannung zu vermindern, einschließlich der Erzeugung amnestischer Effekte, zur Behandlungseinleitung schwerer neurotischer Angstsymptomatik und ausgeprägter Phobien, zur adjuvanten Behandlung schwerer Angst- und Erregungszustände bei Psychosen und Depressionen und zur Behandlung des Status epilepticus.

Das in der erfindungsgemäßen Zusammensetzung enthaltene Lormetazepam (INN) ist ein 7-Chloro-5-(o-chlorophenyl)-1,3-dihydroxy-3-hydroxy-1-methyl-2H-1,4-benzo-diazepin-2-on (WHO) bzw. 9-Chloro-6-(2-chlorophenyl)-4- hydroxy-2-methyl-2,5-diazabicyclo[5.4.0]undeca-5,8,10,12-tetraen-3-on (systema-tischer Name der IUPAC).

Lormetazepam ist eine psychotrope Substanz aus der Klasse der 1,4-Benzodiazepine mit sedierenden und hypnotischen Wirkungen sowie spannungs-, erregungs- und angstdämpfenden Effekten. Darüber hinaus hat Lormetazepam eine zentral muskelrelaxierende und antikonvulsive Wirkung.

Lormetazepam hat eine sehr hohe Affinität zu spezifischen Bindungsstellen im Zentralnervensystem. Diese Bindungsstellen stehen in enger funktioneller Verbindung mit den Rezeptoren des inhibitorischen Neurotransmitters GABA.

Intravenös verabreichte lormetazepamhaltige therapeutische Zusammensetzungen sind seit 1983 in Deutschland zugelassen zur Behandlung von akuten Angstzuständen, zur präoperativen Anxiolyse, zur Prämedikation von Narkosen bei chirurgischen Eingriffen und zur Sedierung in der Intensivmedizin.

Oxazepam (INN) ist ein 9-Chloro-4-hydroxy-6-phenyl-2,5-diazabicyclo[5.4.0]undeca-5,8,10,12-tetraen-3-on (systematischer Name der IUPAC).

Oxazepam ist eine psychotrope Substanz aus der Klasse der 1,4-Benzodiazepine mit angst-, spannungs- und erregungsdämpfenden Eigenschaften sowie sedierenden und hypnotischen Effekten. Darüber hinaus zeigt Oxazepam in hohen Dosen den Muskeltonus dämpfende und antikonvulsive Wirkungen.

Oxazepam bindet mit mittelstarker Affinität an spezifische Rezeptoren im Zentralnervensystem, den Benzodiazepinrezeptoren des GABAergen Transmittersystems. Nach Bindung an den Benzodiazepinrezeptor verstärkt Oxazepam die hemmende Wirkung der GABAergen Übertragung.

Intravenös verabreichte oxazepamhaltige pharmazeutische Zusammensetzungen sind bisher im Markt nicht vorhanden, die Substanz ist jedoch in einer oralen Darreichungsform in vielen Ländern als Arzneimittel zugelassen. In dieser Form ist sie zur symptomatischen Behandlung von akuten und chronischen Angst-, Spannungs- und Erregungszuständen, zur ergänzenden kurzfristigen Therapie schwerer Angst-, Spannungs- und Erregungszustände und zur symptomatischen Behandlung von Durchschlafstörungen zugelassen.

Macrogol 15 Hydroxystearat (Monographiename / Ph.Eur.), mit dem Markennamen Solutol^{®} HS 15, ist ein nichtionisches Lösungsmittel für Injektionslösungen, das aus einer Mischung von überwiegend Mono- und Diestern der 12-Hydroxystearinsäure und Macrogolen besteht (15 Mol Ethylenoxid und 1 Mol 12-Hydroxystearinsäure).

Macrogol 15 Hydroxystearat umfasst Polyglykolmono- und -diester der 12-Hydroxy-stearinsäure (lipophiler Teil) und aus ca. 30% freiem Polyethylenglykol (hydrophiler Teil).

Macrogol 15 Hydroxystearat löst sich in Wasser, Ethanol und 2-Propanol zu einer klaren Lösung, wobei die Löslichkeit in Wasser mit steigender Temperatur abnimmt.

Macrogol 15 Hydroxystearat hat eine hohe chemische Stabilität. Bei längerer Zufuhr von Wärme kann jedoch eine physikalische Trennung in eine flüssige und eine feste Phase auftreten. Diese kann durch nachfolgende Homogenisierung rückgängig gemacht werden.

Wässrige Lösungen von Macrogol 15 Hydroxystearat können bei 121°C autoklaviert werden. Dabei kann es zu einem leichten Abfall des pH-Werts kommen, auch kann dabei eine Phasentrennung auftreten. Letztere kann durch Schütteln der warmen Lösung rückgängig gemacht werden.

Wässrige Lösungen von Macrogol 15 Hydroxystearat können durch Zugabe von in Arzneimitteln üblichen Konservierungsmitteln stabilisiert werden.

Macrogol 15 Hydroxystearat hat neben einem guten Lösungsvermögen, der Autoklavierbarkeit und guter Stabilität in wässrigen Lösungen ein günstiges toxisches Profil, beispielsweise eine geringe hämolytische Aktivität.

Macrogol 15 Hydroxystearat ist für die parenterale Anwendung zugelassen.

Weder Benzodiazepin und/oder Benzodiazepinderivate noch nichtionische Lösungsvermittler weisen eine opiatanaloge Wirkung auf.

In der DE 4039602 A1 werden polymere Verbindungen beschrieben, die als Lösungsvermittler für schwer lösliche Arzneistoffe, zum Einsatz in pharmazeutischen Erzeugnissen zur intravenösen Injektion, verwendet werden können. Hierin wird auch Macrogol 15 Hydroxystearat als nichtionischer Lösungsvermittler genannt.

In der US 2006/0183722 A1 werden ferner lipophile pharmazeutische Substanzen mit schlechter Wasserlöslichkeit und den damit einhergehenden Problemen bei der parenteralen Verabreichung beschrieben. Durch die Verwendungen von nichtionischen Tensiden als Solubilisatoren werden pharmazeutische Erzeugnisse erhalten, die zur parenteralen Verabreichung geeignet sind. Unter den lipophilen Wirkstoffen werden auch Benzodiazepine genannt. Als geeignete Lösungs-vermittler werden nichtionische Solubilisationsmittel, wie Macrogol, erwähnt.

In keiner der genannten Veröffentlichungen wird jedoch auf eine opiatanaloge Wirkung der einzelnen Substanzen oder ihrer Kombination hingewiesen.

Die Aufgabe der Erfindung besteht darin, eine pharmazeutische Zusammensetzung, die eine opiatanaloge Wirkung induziert, bereitzustellen.

Die Aufgabe wird in überraschender Weise durch eine pharmazeutische Zusammensetzung gemäß der beiliegenden Ansprüche gelöst-. In dieser Kombination wird eine opiatanaloge Wirkung induziert, die als Straub'sches Phänomen beschrieben ist.

Der nichtionische Lösungsvermittler ist Macrogol 15 Hydroxystearat.

Gemäß der Erfindung ist die pharma-zeutische Zusammensetzung dadurch gekennzeichnet, dass sie Lormetazepam und/oder ein Derivat davon enthält. Es können auch deren pharma-zeutisch verträgliche Salze eingesetzt werden.

Besonders bevorzugt ist ferner der Einsatz des nichtionischen Lösungsvermittlers einer Menge von 2-45 Gew.-%.

Beim Einsatz von Lorazepam (nicht erfindungsgemäß) wird allgemein der nichtionische Lösungsvermittler bevorzugt in der Menge von 2% bis 10% (W/W), besonders bevorzugt in einer Menge von 3% (W/W) eingesetzt.

Beim Einsatz von Lormetazepam wird allgemein der nichtionische Lösungs-vermittler bevorzugt in der Menge von 3% bis 10% (W/W), besonders bevorzugt in einer Menge von 4% (W/W) eingesetzt.

Beim Einsatz von Oxazepam (nicht erfindungsgemäß) wird allgemein der nichtionische Lösungsvermittler bevorzugt in der Menge von 10% bis 25% (W/W), besonders bevorzugt in einer Menge von 5% (W/W) eingesetzt.

Im Folgenden wird die Induktion des Straub'schen Phänomens durch verschiedene Lösungsmittel und durch Lormetazepam in verschiedenen Lösungsmitteln dargestellt.

| Studie | Nr. | Medikation | Dosis mg/ml | Spezies | Geschlecht | Gewicht 1/g | Gewicht 7/g | Verhalten | Test bestanden | Straub |
|---|---|---|---|---|---|---|---|---|---|---|
| GMF-LOR03tox | 1 | LOR AL | 0,4/1 | Maus NMRI | m | 25 | 29 | normal | ja | nein |
| GMF-LOR03tox | 2 | LOR AL | 0,4/1 | Maus NMRI | m | 25 | 29 | normal | ja | nein |
| GMF-LOR03tox | 3 | LOR AL | 0,4/1 | Maus NMRI | m | 25 | 31 | normal | ja | nein |
| GMF-LOR03tox | 4 | LOR AL | 0,4/1 | Maus NMRI | m | 28 | 29 | normal | ja | nein |
| GMF-LOR03tox | 5 | LOR AL | 0,4/1 | Maus NMRI | m | 24 | 31 | normal | ja | nein |
| | | | | | | | | | | |
| GMF-LOR03tox | 1 | LOR IL | 0,4/1 | Maus NMRI | m | 25 | 31 | normal | ja | nein |
| GMF-LOR03tox | 2 | LOR IL | 0,4/1 | Maus NMRI | m | 26 | 31 | normal | ja | nein |
| GMF-LOR03tox | 3 | LOR IL | 0,4/1 | Maus NMRI | m | 26 | 29 | normal | ja | nein |
| GMF-LOR03tox | 4 | LOR IL | 0,4/1 | Maus NMRI | m | 27 | 32 | normal | ja | nein |
| GMF-LOR03tox | 5 | LOR IL | 0,4/1 | Maus NMRI | m | 25 | 29 | normal | ja | nein |
| | | | | | | | | | | |
| GMF-LOR03tox | 1 | LOR LF | 0,4/1 | Maus NMRI | m | 24 | 29 | normal | ja | nein |
| GMF-LOR03tox | 2 | LOR LF | 0,4/1 | Maus NMRI | m | 25 | 31 | normal | ja | nein |
| GMF-LOR03tox | 3 | LOR LF | 0,4/1 | Maus NMRI | m | 24 | 31 | normal | ja | nein |
| GMF-LOR03tox | 4 | LOR LF | 0,4/1 | Maus NMRI | m | 26 | 32 | normal | ja | nein |
| GMF-LOR03tox | 5 | LOR LF | 0,4/1 | Maus NMRI | m | 25 | 29 | normal | ja | nein |
| | | | | | | | | | | |
| GMF-LOR03tox | 1 | LOR LV | 0,4/1 | Maus NMRI | m | 26 | 29 | normal | ja | nein |
| GMF-LOR03tox | 2 | LOR LV | 0,4/1 | Maus NMRI | m | 25 | 32 | normal | ja | nein |
| GMF-LOR03tox | 3 | LOR LV | 0,4/1 | Maus NMRI | m | 26 | 31 | normal | ja | nein |
| GMF-LOR03tox | 4 | LOR LV | 0,4/1 | Maus NMRI | m | 26 | 29 | normal | ja | nein |
| GMF-LOR03tox | 5 | LOR LV | 0,4/1 | Maus NMRI | m | 25 | 29 | normal | ja | nein |
| | | | | | | | | | | |
| GMF-LOR03tox | 1 | LOR SL | 0,4/1 | Maus NMRI | m | 20 | 26 | normal | ja | nein |
| GMF-LOR03tox | 2 | LOR SL | 0,4/1 | Maus NMRI | m | 21 | 26 | normal | ja | nein |
| GMF-LOR03tox | 3 | LOR SL | 0,4/1 | Maus NMRI | m | 22 | 27 | normal | ja | nein |
| GMF-LOR03tox | 4 | LOR SL | 0,4/1 | Maus NMRI | m | 21 | 29 | normal | ja | nein |
| GMF-LOR03tox | 5 | LOR SL | 0,4/1 | Maus NMRI | m | 20 | 28 | normal | ja | nein |
| GMF-LOR04tox | 1 | SO HS 15 | 40/1 | Maus NMRI | m | 21 | 27 | normal | ja | nein |
| GMF-LOR04tox | 2 | SO HS 15 | 40/1 | Maus NMRI | m | 20 | 28 | normal | ja | nein |
| GMF-LOR04tox | 3 | SO HS 15 | 40/1 | Maus NMRI | m | 21 | 29 | normal | ja | nein |
| GMF-LOR04tox | 4 | SO HS 15 | 40/1 | Maus NMRI | m | 21 | 27 | normal | ja | nein |
| GMF-LOR04tox | 5 | SO HS 15 | 40/1 | Maus NMRI | m | 20 | 28 | normal | ja | nein |
| | | | | | | | | | | |
| GMF-LOR04tox | 1 | SO HS 15 | 400/1 | Maus NMRI | m | 22 | 30 | normal | ja | nein |
| GMF-LOR04tox | 2 | SO HS 15 | 400/1 | Maus NMRI | m | 20 | 28 | normal | ja | nein |
| GMF-LOR04tox | 3 | SO HS 15 | 400/1 | Maus NMRI | m | 23 | 30 | normal | ja | nein |
| GMF-LOR04tox | 4 | SO HS 15 | 400/1 | Maus NMRI | m | 21 | 29 | normal | ja | nein |
| GMF-LOR04tox | 5 | SO HS 15 | 400/1 | Maus NMRI | m | 20 | 28 | normal | ja | nein |
| | | | | | | | | | | |
| GMF-LOR04tox | 1 | LOR WL | 0,2/1 | Maus NMRI | m | 19 | 25 | normal | ja | ja |
| GMF-LOR04tox | 2 | LOR WL | 0,2/1 | Maus NMRI | m | 20 | 27 | normal | ja | ja |
| GMF-LOR04tox | 3 | LOR WL | 0,2/1 | Maus NMRI | m | 20 | 26 | normal | ja | ja |
| GMF-LOR04tox | 4 | LOR WL | 0,2/1 | Maus NMRI | m | 19 | 26 | normal | ja | ja |
| GMF-LOR04tox | 5 | LOR WL | 0,2/1 | Maus NMRI | m | 21 | 29 | normal | ja | ja |
| | | | | | | | | | | |
| GMF-LOR04tox | 1 | LOR WL | 0,2/1 | Maus NMRI | w | 19 | 25 | normal | ja | ja |
| GWF-LOR04tox | 2 | LOR WL | 0,2/1 | Maus NMRI | w | 18 | 24 | normal | ja | ja |
| GWF-LOR04tox | 3 | LOR WL | 0,2/1 | Maus NMRI | w | 18 | 26 | normal | ja | ja |
| GWF-LOR04tox | 4 | LORWL | 0,2/1 | Maus NMRI | w | 19 | 25 | normal | ja | ja |
| GWF-LOR04tox | 5 | LOR WL | 0,2/1 | Maus NMRI | w | 18 | 27 | normal | ja | ja |
| | | | | | | | | | | |
| GMF-PG50tox | 1 | PG 50% | 125/0,25 | Maus NMRI | m | 20 | 26 | normal | ja | nein |
| GMF-PG50tox | 2 | PG 50% | 125/0,25 | Maus NMRI | m | 19 | 26 | normal | ja | nein |
| GMF-PG50tox | 3 | PG 50% | 125/0,25 | Maus NMRI | m | 20 | 26 | normal | ja | nein |
| GMF-PG50tox | 4 | PG 50% | 125/0,25 | Maus NMRI | m | 21 | 27 | normal | ja | nein |
| GMF-PG50tox | 5 | PG 50% | 125/0,25 | Maus NMRI | m | 20 | 25 | normal | ja | nein |
| GMF-LOR04tox | 1 | NOCTAMID | 0,05/1 | Maus NMRI | m | 18 | 24 | normal | ja | nein |
| GMF-LOR04tox | 2 | NOCTAMID | 0,05/1 | Maus NMRI | m | 20 | 25 | normal | ja | nein |
| GMF-LOR04tox | 3 | NOCTAMID | 0,05/1 | Maus NMRI | m | 20 | 26 | normal | ja | nein |
| GMF-LOR04tox | 4 | NOCTAMID | 0,05/1 | Maus NMRI | m | 19 | 26 | normal | ja | nein |
| GMF-LOR04tox | 5 | NOCTAMID | 0,05/1 | Maus NMRI | m | 20 | 27 | normal | ja | nein |
| | | | | | | | | | | |
| GMF-LOR04tox | 1 | LOR WL | 0,4/1 | Maus NMRI | m | 20 | 27 | normal | ja | ja |
| GMF-LOR04tox | 2 | LOR WL | 0,4/1 | Maus NMRI | m | 21 | 28 | normal | ja | ja |
| GMF-LOR04tox | 3 | LOR WL | 0,4/1 | Maus NMRI | m | 20 | 26 | normal | ja | ja |
| GMF-LOR04tox | 4 | LOR WL | 0,4/1 | Maus NMRI | m | 19 | 26 | normal | ja | ja |
| GMF-LOR04tox | 5 | LOR WL | 0,4/1 | Maus NMRI | m | 20 | 27 | normal | ja | ja |
| | | | | | | | | | | |
| GMF-LOR04tox | 1 | LOR WL | 0,4/1 | Maus NMRI | w | 19 | 26 | normal | ja | ja |
| GMF-LOR04tox | 2 | LOR WL | 0,4/1 | Maus NMRI | w | 18 | 26 | normal | ja | ja |
| GMF-LOR04tox | 3 | LORWL | 0,4/1 | Maus NMRI | w | 20 | 27 | normal | ja | ja |
| GMF-LOR04tox | 4 | LOR WL | 0,4/1 | Maus NMRI | w | 19 | 27 | normal | ja | ja |
| GMF-LOR04tox | 5 | LOR WL | 0,4/1 | Maus NMRI | w | 19 | 26 | normal | ja | ja |

| | | | | | | | | | | |
|---|---|---|---|---|---|---|---|---|---|---|
| Abkürzungen: LOR AL: Lormetazepam / Abbolipid LOR IL: Lormetazepam / Intralipid LOR LF: Lormetazepam / Lipofundin LOR SL: Lormetazepam / Salvilipid SO HS 15: Macrogol 15 Hydroxystearat LOR WL: Lormetazepam / Macrogol 15 Hydroxystearat NOCTAMID: Lormetazepam / Propylenglykol 50 % PG 50%: Propylenglykol 50 % NMRI: Naval Medical Research Institute w: weiblich m: männlich Gewicht 1/g: Gewicht in Gramm an Tag 1 vor der Verabreichung der jeweiligen Medikation Gewicht 7/g: Gewicht in Gramm an Tag 7 (Ende der Studie) Straub: Induktion des Straub'schen Phänomens | | | | | | | | | | |

Neben dem Vorliegen der erfindungsgemäßen pharmazeutischen Zusammensetzung in Form einer Lösung oder in Form eines Gels ist auch eine andere Zubereitungsform möglich.

Bei Versuchen mit in Macrogol 15 Hydroxystearat gelösten Benzodiazepinen kam es überraschenderweise kurz nach Injektion der Lösung bei allen behandelten Mäusen zum Auftreten eines sog. Straub'schen Phänomens. Dieses hielt an, bis die Tiere medikationsbedingt einschliefen.
Lorazepam: 0,03-0,05 mg in 0,2 ml i.v./Maus (Gewicht ca. 20 g) (nicht erfindungsgemäß)
Lormetazepam: 0,03-0,05 mg in 0,2 ml i.v. / Maus (Gewicht ca. 20 g)
Oxazepam: 0,16-0,24 mg in 0,2 ml i.v./Maus (Gewicht ca. 20 g) (nicht erfindungsgemäß)

Das Straub'sche Phänomen, auch Mäuseschwanzphänomen bzw. Straub Tail Reaction (STR) genannt, zeigt sich in einer S-förmigen Dorsalflexion des Schwanzes und einer Protrusion des Perineums, die durch eine Kontraktion des musculus sacro-coccygeus hervorgerufen wird.

Das Auftreten dieses Phänomens war deshalb unerwartet, weil es bisher nur nach Injektion von Morphin oder Morphin-Analoga beobachtet worden ist. Deshalb diente das Straub'sche Phänomen früher als sicherer qualitativer und semiquantitativer Nachweis einer peripheren Morphinwirkung.

Die Morphinreaktion wird durch Stimulation der motorischen Innervation auf der Ebene des lumbosacralen Rückenmarks hervorgerufen. Morphin wirkt auf lumbosacrale Nervenzellen und über das spinale ventrale Hörn absteigende Nervenendigungen.

Es ist besonders darauf hinzuweisen, dass das Straub'sche Phänomen weder bei der Applikation des nichtionischen Lösungsvermittlers allein noch bei der Applikation von Benzodiazepin und/oder Benzodiazepinderivaten mit anderen Lösungsmitteln und insbesondere nicht bei der Applikation von Benzodiazepin und/oder Benzodiazepinderivaten mit einer Öl-in-Wasser Emulsion als Lösungsmittel (EP 0682943 A1) sondern nur bei der oben genannten erfindungsgemäßen Kombination auftritt.

Das Mäuseschwanz-Phänomen wird u.a. bei Zarrindast MR, Alaei-Nia K, Shafizadeh M, On the mechanism of tolerance to morphine-induced Straub tail reaction in mice, Pharmacol Biochem Behav. 2001 Jul-Aug; 69(3-4): 419-24 und Zarrindast MR, Ghadimi M, Ramezani-Tehrani B, Sahebgharani M., EFFECT OF GABA RECEPTOR AGONISTS OR ANTAGONISTS ON MORPHINE-INDUCED STRAUB TAIL IN MICE, International Journal of Neuroscience 2006, 116: 963-973 beschrieben.

Die erfindungsgemäße pharmazeutische Zusammensetzung löst die gestellte Aufgabe in hervorragender Weise.

Die der Erfindung zugrunde liegende überraschende Beobachtung bedeutet, dass der jeweilige Wirkstoff in Kombination mit dem nichtionischen Lösungsvermittler ein über das von Benzodiazepin und/oder Benzodiazepinderivaten hinausgehendes Wirkspektrum besitzt.

Die pharmazeutische Zusammensetzung ist insbesondere für Anwendungen in der Anästhesie, Intensivmedizin, Schmerztherapie oder Neurologie/Psychiatrie besonders gut geeignet.

Die Erfindung betrifft daher auch die erfindungsgemäße pharmazeutische Zusammensetzung zur Verwendung als Arzneimittel zur Induktion einer opiatanalogen Wirkung, die als Straub'sches Phänomen beschrieben ist, insbesondere zur Anwendung in der Anästhesie, Intensivmedizin, Schmerztherapie und Neurologie/Psychiatrie.

Die pharmazeutische Zusammensetzung ist in entsprechender Weise auch zur Anwendung beim tierischen Körper geeignet.

Allgemeine Herstellung einer Injektions/ Infusionslösung

Gemäß einem Herstellungsverfahren lässt sich die Benzodiazepin und/oder Benzodiazepinderivate enthaltende pharmazeutische Zusammensetzung wie folgt herstellen.

Der Lösungsvermittler Macrogol 15 Hydroxystearat wird in einem Behälter mit Rührer auf eine Temperatur von 40-60°C temperiert. Dann wird der darin leicht lösliche Wirkstoff zugegeben und unter Rühren gelöst. Nach 15 bis 30 min bei einer Temperatur von 50°C (± 10°C) liegt eine klare Lösung vor.

Die klare Lösung wird anschließend in 50-60°C warmes Wasser für Injektionszwecke eingerührt. Zur Verhinderung von Entmischung bei der Zugabe sollten beide Phasen vorzugsweise eine Temperatur von 55 ± 5°C aufweisen. Anschließend wird der Ansatz unter Rühren auf 20-30°C abgekühlt. Nach Abfüllung in Ampullen oder Vialen kann die Injektionslösung autoklaviert werden.

Entsprechend betrifft die Erfindung in einem weiteren Aspekt ein Verfahren zum Herstellen einer Injektions- oder Infusionslösung, umfassend Lormetazepam oder ein Derivat oder pharmazeutisch verträgliche Salze davon sowie Macrogol 15 Hydroxystearat als nichtionischem Lösungsvermittler gemäß Anspruch 7.

Die Erfindung wird nachfolgend anhand von Beispielen näher erläutert.

### Beispiel 1 (nicht erfindungsgemäß)

400 g Macrogol 15 Hydroxystearat werden geschmolzen. Bei einer Temperatur von 50°C werden 2,0 g Lorazepam zugegeben und unter Rühren gelöst. Die klare Lösung wird unter Rühren zu vorher auf 50°C erwärmtes Wasser für Injektions-zwecke (Wfl) Ph.Eur. (ca. 8 Liter) gegeben und gemischt. Anschließend wird mit Wfl auf genau 10 Liter verdünnt, um eine Wirkstoffkonzentration von 0,2 mg/ml zu erhalten.

Die Lösung wird abgekühlt und sterilfiltriert. Anschließend werden je 10 ml der Lösung in Glas-Ampullen abgefüllt und bei 121°C nach Ph. Eur. sterilisiert. Jede so hergestellte Ampulle enthält somit die gewünschte therapeutische Dosis 2 mg Wirkstoff.

### Beispiel 2 (nicht erfindungsgemäß)

600 g Macrogol 15 Hydroxystearat werden geschmolzen. Bei einer Temperatur von 50°C werden 2,0 g Lorazepam zugegeben und unter Rühren gelöst. Die klare Lösung wird unter Rühren zu vorher auf 50°C erwärmtes Wasser für Injektionszwecke (Wfl) Ph.Eur. (ca. 4 Liter) gegeben und gemischt. Anschließend wird mit Wfl auf genau 5 Liter verdünnt, um eine Wirkstoffkonzentration von 0,4 mg/ml zu erhalten.
Die Lösung wird abgekühlt und sterilfiltriert. Anschließend werden je 5 ml der Lösung in Glas-Ampullen abgefüllt und bei 121°C nach Ph. Eur. sterilisiert. Jede so hergestellte Ampulle enthält somit die gewünschte therapeutische Dosis 2 mg Wirkstoff.

### Beispiel 3 (nicht erfindungsgemäß)

200 g Macrogol 15 Hydroxystearat werden geschmolzen. Bei einer Temperatur von 50°C werden 2,0 g Lorazepam zugegeben und unter Rühren gelöst. Die klare Lösung wird unter Rühren zu vorher auf 50°C erwärmtes Wasser für Injektionszwecke (Wfl) Ph.Eur. (ca. 8 Liter) gegeben und gemischt. Anschließend wird mit Wfl auf genau 10 Liter verdünnt, um eine Wirkstoffkonzentration von 0,2 mg/ml zu erhalten.

Die Lösung wird abgekühlt und sterilfiltriert. Anschließend werden je 10 ml der Lösung in Glas-Ampullen abgefüllt und bei 121°C nach Ph. Eur. sterilisiert. Jede so hergestellte Ampulle enthält somit die gewünschte therapeutische Dosis 2 mg Wirkstoff.

### Beispiel 4 (nicht erfindungsgemäß)

500 g Macrogol 15 Hydroxystearat werden geschmolzen. Bei einer Temperatur von 50°C werden 2,0 g Lorazepam zugegeben und unter Rühren gelöst. Die klare Lösung wird unter Rühren zu vorher auf 50°C erwärmtes Wasser für Injektionszwecke (Wfl) Ph.Eur. (ca. 4 Liter) gegeben und gemischt. Anschließend wird mit Wfl auf genau 5 Liter verdünnt, um eine Wirkstoffkonzentration von 0,4 mg/ml zu erhalten. Die Lösung wird abgekühlt und sterilfiltriert. Anschließend werden je 5 ml der Lösung in Glas-Ampullen abgefüllt und bei 121 °C nach Ph.Eur. sterilisiert. Jede so hergestellte Ampulle enthält somit die gewünschte therapeutische Dosis 2 mg Wirkstoff.

### Beispiel 5

400 g Macrogol 15 Hydroxystearat werden geschmolzen. Bei einer Temperatur von 50°C werden 2,0 g Lormetazepam zugegeben und unter Rühren gelöst. Die klare Lösung wird unter Rühren zu vorher auf 50°C erwärmtes Wasser für Injektionszwecke (Wfl) Ph.Eur. (ca. 8 Liter) gegeben und gemischt. Anschließend wird mit Wfl auf genau 10 Liter verdünnt, um eine Wirkstoffkonzentration von 0,2 mg/ml zu erhalten.

Die Lösung wird abgekühlt und sterilfiltriert. Anschließend werden je 10 ml der Lösung in Glas-Ampullen abgefüllt und bei 121°C nach Ph. Eur. sterilisiert. Jede so hergestellte Ampulle enthält somit die gewünschte therapeutische Dosis 2 mg Wirkstoff.

### Beispiel 6

600 g Macrogol 15 Hydroxystearat werden geschmolzen. Bei einer Temperatur von 50°C werden 2,0 g Lormetazepam zugegeben und unter Rühren gelöst. Die klare Lösung wird unter Rühren zu vorher auf 50°C erwärmtes Wasser für Injektionszwecke (Wfl) Ph.Eur. (ca. 4 Liter) gegeben und gemischt. Anschließend wird mit Wfl auf genau 5 Liter verdünnt, um eine Wirkstoffkonzentration von 0,4 mg/ml zu erhalten.

Die Lösung wird abgekühlt und sterilfiltriert. Anschließend werden je 5 ml der Lösung in Glas-Ampullen abgefüllt und bei 121°C nach Ph. Eur. sterilisiert. Jede so hergestellte Ampulle enthält somit die gewünschte therapeutische Dosis 2 mg Wirkstoff.

### Beispiel 7

200 g Macrogol 15 Hydroxystearat werden geschmolzen. Bei einer Temperatur von 50°C werden 2,0 g Lormetazepam zugegeben und unter Rühren gelöst. Die klare Lösung wird unter Rühren zu vorher auf 50°C erwärmtes Wasser für Injektionszwecke (Wfl) Ph.Eur. (ca. 8 Liter) gegeben und gemischt. Anschließend wird mit Wfl auf genau 10 Liter verdünnt, um eine Wirkstoffkonzentration von 0,2 mg/ml zu erhalten.

Die Lösung wird abgekühlt und sterilfiltriert. Anschließend werden je 10 ml der Lösung in Glas-Ampullen abgefüllt und bei 121°C nach Ph. Eur. sterilisiert. Jede so hergestellte Ampulle enthält somit die gewünschte therapeutische Dosis 2 mg Wirkstoff.

### Beispiel 8 (nicht erfindungsgemäß)

500 g Macrogol 15 Hydroxystearat werden geschmolzen. Bei einer Temperatur von 50°C werden 2,0 g Lorazepam zugegeben und unter Rühren gelöst. Die klare Lösung wird unter Rühren zu vorher auf 50°C erwärmtes Wasser für Injektionszwecke (Wfl) Ph.Eur. (ca. 4 Liter) gegeben und gemischt. Anschließend wird mit Wfl auf genau 5 Liter verdünnt, um eine Wirkstoffkonzentration von 0,4 mg/ml zu erhalten. Die Lösung wird abgekühlt und sterilfiltriert. Anschließend werden je 5 ml der Lösung in Glas-Ampullen abgefüllt und bei 121°C nach Ph.Eur. sterilisiert. Jede so hergestellte Ampulle enthält somit die gewünschte therapeutische Dosis 2 mg Wirkstoff.

### Beispiel 9 (nicht erfindungsgemäß)

1500 g Macrogol 15 Hydroxystearat werden geschmolzen. Bei einer Temperatur von 50°C werden 10,0 g Oxazepam zugegeben und unter Rühren gelöst. Die klare Lösung wird unter Rühren zu vorher auf 50°C erwärmtes Wasser für Injektionszwecke (Wfl) Ph.Eur. (ca. 7 Liter) gegeben und gemischt. Anschließend wird mit Wfl auf genau 10 Liter verdünnt, um eine Wirkstoffkonzentration von 1,0 mg/ml zu erhalten.

Die Lösung wird abgekühlt und sterilfiltriert. Anschließend werden je 10 ml der Lösung in Glas-Ampullen abgefüllt und bei 121°C nach Ph. Eur. sterilisiert. Jede so hergestellte Ampulle enthält somit die gewünschte therapeutische Dosis 10 mg Wirkstoff.

### Beispiel 10 (nicht erfindungsgemäß)

2000 g Macrogol 15 Hydroxystearat werden geschmolzen. Bei einer Temperatur von 50°C werden 10,0 g Oxazepam zugegeben und unter Rühren gelöst. Die klare Lösung wird unter Rühren zu vorher auf 50°C erwärmtes Wasser für Injektionszwecke (WFI) Ph.Eur. (3,5 Liter) gegeben und gemischt. Anschließend wird mit Wfl auf genau 5 Liter verdünnt, um eine Wirkstoffkonzentration von 2,0 mg/ml zu erhalten.

Die Lösung wird abgekühlt und sterilfiltriert. Anschließend werden je 5 ml der Lösung in Glas-Ampullen abgefüllt und bei 121°C nach Ph. Eur. sterilisiert. Jede so hergestellte Ampulle enthält somit die gewünschte therapeutische Dosis 10 mg Wirkstoff.

### Beispiel 11 (nicht erfindungsgemäß)

3000 g Macrogol 15 Hydroxystearat werden geschmolzen. Bei einer Temperatur von 50°C werden 20,0 g Oxazepam zugegeben und unter Rühren gelöst. Die klare Lösung wird unter Rühren zu vorher auf 50°C erwärmtes Wasser für Injektionszwecke (WFI) Ph.Eur. (ca. 14 Liter) gegeben und gemischt. Anschließend wird mit Wfl auf genau 20 Liter verdünnt, um eine Wirkstoffkonzentration von 1,0 mg/ml zu erhalten.

Die Lösung wird abgekühlt und sterilfiltriert. Anschließend werden je 20 ml der Lösung in Glas-Ampullen abgefüllt und bei 121 °C nach Ph. Eur, sterilisiert. Jede so hergestellte Ampulle enthält somit die gewünschte therapeutische Dosis 20 mg Wirkstoff.

### Beispiel 12 (nicht erfindungsgemäß)

2500 g Macrogol 15 Hydroxystearat werden geschmolzen. Bei einer Temperatur von 50°C werden 20,0 g Oxazepam zugegeben und unter Rühren gelöst. Die klare Lösung wird unter Rühren zu vorher auf 50°C erwärmtes Wasser für Injektionszwecke (Wfl) Ph. Eur. (ca. 7 Liter) gegeben und gemischt. Anschließend wird mit Wfl auf genau 10 Liter verdünnt, um eine Wirkstoffkonzentration von 2,0 mg/ml zu erhalten.

Die Lösung wird abgekühlt und sterilfiltriert. Anschließend werden je 10 ml der Lösung in Glas-Ampullen abgefüllt und bei 121°C nach Ph. Eur. sterilisiert. Jede so hergestellte Ampulle enthält somit die gewünschte therapeutische Dosis 20 mg Wirkstoff.

### Beispiel 13 (nicht erfindungsgemäß)

3000 g Macrogol 15 Hydroxystearat werden geschmolzen. Bei einer Temperatur von 50°C werden 30,0 g Oxazepam zugegeben und unter Rühren gelöst. Die klare Lösung wird unter Rühren zu vorher auf 50°C erwärmtes Wasser für Injektionszwecke Ph. Eur. (ca. 14 Liter) gegeben und gemischt. Anschließend wird mit Wfl auf genau 20 Liter verdünnt, um eine Wirkstoffkonzentration von 1,5 mg/ml zu erhalten.

Die Lösung wird abgekühlt und sterilfiltriert. Anschließend werden je 20 ml der Lösung in Glas-Ampullen abgefüllt und bei 121 °C nach Ph. Eur. sterilisiert. Jede so hergestellte Ampulle enthält somit die gewünschte therapeutische Dosis 30 mg Wirkstoff.

### Beispiel 14 (nicht erfindungsgemäß)

7500 g Macrogol 15 Hydroxystearat werden geschmolzen. Bei einer Temperatur von 50°C werden 50,0 g Oxazepam zugegeben und unter Rühren gelöst. Die klare Lösung wird unter Rühren zu vorher auf 50°C erwärmtes Wasser für Injektionszwecke (Wfl) Ph.Eur. (ca. 40 Liter) gegeben und gemischt. Anschließend wird mit Wfl auf genau 50 Liter verdünnt, um eine Wirkstoffkonzentration von 1,0 mg/ml zu erhalten.

Die Lösung wird abgekühlt und sterilfiltriert. Anschließend werden je 50 ml der Lösung in Glas-Vialen abgefüllt und bei 121°C nach Ph. Eur, sterilisiert. Jedes so hergestellte Vial enthält somit die gewünschte therapeutische Dosis 50 mg Wirkstoff.

## Patentansprüche

1. Pharmazeutische Zusammensetzung, umfassend Lormetazepam oder ein Derivat oder pharmazeutisch verträgliche Salze davon sowie Macrogol 15 Hydroxystearat als nichtionischem Lösungsvermittler.

2. Pharmazeutische Zusammensetzung nach Anspruch 1, wobei der nichtionische Lösungsvermittler in einer Menge von 2-45 Gew.-% enthalten ist.

3. Pharmazeutische Zusammensetzung nach Anspruch 1 oder 2, wobei sie in Form einer Lösung vorliegt.

4. Pharmazeutische Zusammensetzung nach Anspruch 1 oder 2, wobei sie in Form eines Gels vorliegt.

5. Pharmazeutische Zusammensetzung nach Anspruch 3, wobei die Zusammensetzung aus Lormetazepam oder einem Derivat oder pharmazeutisch verträglichen Salzen davon, Macrogol 15 Hydroxystearat und Wasser besteht.

6. Pharmazeutische Zusammensetzung nach einem der Ansprüche 1 bis 5 zur Verwendung als Arzneimittel zur Induktion einer opiatanalogen Wirkung, die als Straub'sches Phänomen beschrieben ist, insbesondere zur Anwendung in der Anästhesie, Intensivmedizin, Schmerztherapie oder Neurologie/Psychiatrie.

7. Verfahren zum Herstellen einer Injektions- oder Infusionslösung, umfassend Lormetazepam oder ein Derivat oder pharmazeutisch verträgliche Salze davon sowie Macrogol 15 Hydroxystearat als nichtionischem Lösungsvermittler, wobei das Verfahren die folgenden Schritte umfasst:
Temperieren von Macrogol 15 Hydroxystearat auf 40-60 °C,
Zugabe von Lormetazepam oder einem Derivat oder pharmazeutisch verträglichen Salzen davon und Lösen desselben bzw. derselben unter Rühren,
Einrühren der so erhaltenen klaren Lösung in 50-60 °C warmes Wasser für Injektionszwecke, und
Abkühlen des Ansatzes unter Rühren auf 20-30 °C.

## Claims

1. A pharmaceutical composition, comprising lormetazepam or a derivative or pharmaceutically acceptable salts thereof, and macrogol-15-hydroxystearate as non-ionic solubilizer.

2. The pharmaceutical composition according to claim 1, wherein the non-ionic solubilizer is present in an amount of 2-45 wt.%.

3. The pharmaceutical composition according to claim 1 or 2, wherein the composition is in form of a solution.

4. The pharmaceutical composition according to claim 1 or 2, wherein the composition is in form of a gel.

5. The pharmaceutical composition according to claim 3, wherein the composition consists of lormetazepam or a derivative or pharmaceutically acceptable salts thereof, macrogol-15-hydroxystearate, and water.

6. A pharmaceutical composition according to any one of claims 1 to 5 for use as a medicament for inducing an opiate-like effect, described as Straub's phenomenon, in particular for use in anesthesia, intensive care medicine, pain therapy, or neurology/psychiatry.

7. A method for preparing an injection solution or an infusion solution comprising lormetazepam or a derivative or pharmaceutically acceptable salts thereof, and macrogol-15-hydroxystearate as non-ionic solubilizer, wherein the method comprises the following steps:
regulating macrogol-15-hydroxystearate at a temperature of 40 to 60 °C,
adding lormetazepam or a derivative or pharmaceutically acceptable salts thereof and dissolving it or them while stirring,
stirring the obtained clear solution into water suitable for injection having a temperature of 50 to 60 °C, and
cooling the mixture to 20 to 30 °C while stirring.

## Revendications

1. Composition pharmaceutique comprenant de la lormétazépam, ou un dérivé ou des sels pharmaceutiquement compatibles de celle-ci, ainsi que de l'hydroxystéarate de macrogol 15 servant d'agent de solubilisation non ionique.

2. Composition pharmaceutique selon la revendication 1, l'agent de solubilisation non-ionique étant contenu dans une quantité comprise entre 2 et 45 % en poids.

3. Composition pharmaceutique selon les revendications 1 ou 2, se présentant sous forme d'une solution.

4. Composition pharmaceutique selon les revendications 1 ou 2, se présentant sous forme d'un gel.

5. Composition pharmaceutique selon la revendication 3, la composition étant constituée de lormétazépam, ou d'un dérivé ou de sels pharmaceutiquement compatibles de celle-ci, d'hydroxystéarate de macrogol 15, et d'eau.

6. Composition pharmaceutique selon l'une des revendications 1 à 5, pour une utilisation comme médicament destiné à induire un effet qui est analogue aux opiacés et qui est décrit comme phénomène de Straub, notamment en vue d'une application en anesthésie, en soins intensifs, en thérapie de la douleur ou en neurologie/psychiatrie.

7. Procédé de préparation d'une solution injectable ou destinée à la perfusion laquelle comprend de la lormétazépam, ou un dérivé ou des sels pharmaceutiquement compatibles de celle-ci, ainsi que de l'hydroxystéarate de macrogol 15 servant d'agent de solubilisation non ionique, ledit procédé comprenant les étapes consistant à :
thermoréguler l'hydroxystéarate de macrogol 15 à une température comprise entre 40 et 60 °C,
ajouter de la lormétazépam, ou un dérivé ou des sels pharmaceutiquement compatibles de celle-ci, pour ensuite la/le/les dissoudre sous agitation,
ajouter la solution limpide ainsi obtenue à de l'eau pour solutions injectables, maintenue sous agitation à une température comprise entre 50 et 60 °C, et
refroidir le mélange obtenu, sous agitation, à une température comprise entre 20 et 30 °C.
